Europäisches Patentamt

(19) European Patent Office

Office européen des brevets

(11) EP 0 609 112 B1

# (12) FASCICULE DE BREVET EUROPEEN

(45) Date de publication et mention
de la délivrance du brevet:
**15.07.1998 Bulletin 1998/29**

(21) Numéro de dépôt: **94400080.1**

(22) Date de dépôt: **12.01.1994**

(51) Int. Cl.⁶: $B01J\ 29/40$, $B01J\ 29/44$, $B01J\ 29/06$

(54) **Catalyseur à base de zéolithe MFI modifiée et son utilisation pour l'isomérisation d'une coupe C8 aromatique**

Katalysator aufgrund modifizierter MFI Zeolite und seine Verwendung für die Isomerisierung einer aromatischen C8 Fraktion

Catalyst containing a modified MFI zeolite and its use in the isomerisation of an aromatic C8 fraction

(84) Etats contractants désignés:
**DE ES IT NL**

(30) Priorité: **28.01.1993 FR 9301016**

(43) Date de publication de la demande:
**03.08.1994 Bulletin 1994/31**

(73) Titulaire:
**INSTITUT FRANCAIS DU PETROLE
92502 Rueil-Malmaison (FR)**

(72) Inventeurs:
  • **Benazzi, Eric
    F-78360 Montesson (FR)**
  • **Da Silva, Joao Miguel
    PT-2725 Mem Martins (PT)**
  • **Joly, Jean-François
    F-75003 Paris (FR)**

  • **Gomez Ribeiro, Maria Filipa
    PT-2700 Amadora (PT)**

(74) Mandataire: **Andreeff, François
    INSTITUT FRANCAIS DU PETROLE
    4, avenue de Bois-Préau
    92502 Rueil-Malmaison (FR)**

(56) Documents cités:
**EP-A- 0 279 568**          **EP-A- 0 350 367**
**EP-A- 0 474 536**          **US-A- 3 856 872**
**US-A- 4 098 836**          **US-A- 5 028 573**
**US-A- 5 080 878**

Remarques:
Le dossier contient des informations techniques présentées postérieurement au dépôt de la demande et ne figurant pas dans le présent fascicule.

**Description**

La présente invention concerne un catalyseur de type alumino-silicate comprenant une zéolithe MFI dont on a modifié la sélectivité et/ou les propriétés catalytiques par désalumination de la surface externe de ses cristaux, et comprenant au moins un métal du groupe VIII de la classification périodique des éléments (Handbook of Chemistry and Physics, 65$^{ieme}$ édition, 1984-85), et une matrice. Ledit catalyseur est utilisé dans les réactions d'isomérisation des hydrocarbures C$_8$ aromatiques. L'invention concerne également le procédé de préparation dudit catalyseur . En général, la modification de la surface externe des cristaux de la zéolithe s'effectue par au moins un traitement de la MFI par au moins une solution d'un sel de l'acide fluorosilicique. De préférence, le sel choisi ne conduit pas à la formation de sels d'aluminium insolubles dans l'eau.

Actuellement, les catalyseurs utilisés industriellement dans les réactions d'isomérisation de coupes C$_8$ aromatiques sont essentiellement à base de zéolithe ZSM5 de type structural MFI, seule ou en mélange avec d'autres zéolithes. Ces catalyseurs sont notamment décrits dans les brevets USP 3856872, USP 4098836, USP4159282, USP-4467129, USP-4482773, EP-B-138617 et USP 5028573.

L'intérêt de la zéolithe ZSM5 réside dans sa sélectivité de forme, qui conduit à une sélectivité intéressante en para-xylène et aussi dans sa sélectivité vis-à-vis des réactions secondaires indésirables de dismutation qui restent à un niveau plus faible que ceux enregistrés sur d'autres zéolithes à ouvertures de pores plus larges. En effet, des zéolithes à ouverture de pores plus larges 12MR (ouverture à 12 atomes d'oxygène) ont aussi été utilisées, telle que la mordénite. Les catalyseurs à base de mordénite sont notamment décrits dans les brevets USP-4723051, USP-4665258 et FR-A-2477903. Cependant, ces zéolithes ne possèdent pas de propriétés de sélectivité géométrique particulières. Ceci se traduit, quelque soit leur rapport Si/Al, par des sélectivités en para-xylène plus faibles que celles obtenues pour la zéolithe ZSM5 et surtout par des productions de triméthylbenzènes très importantes. La production de triméthylbenzènes par dismutation est en effet favorisée dans la mordénite dont le système microporeux est plus ouvert que celui de la ZSM5: les ouvertures sont à 12 oxygènes au lieu de 10 pour la ZSM5.

La demanderesse a découvert que, de façon surprenante, il est possible en réalisant au moins un traitement de désalumination d'une MFI par au moins une solution de fluorosilicate d'un cation et en particulier au moins une solution d'hexafluorosilicate d'ammonium, d'obtenir des catalyseurs actifs et sélectifs pour la réaction d'isomérisation des C$_8$ aromatiques. On obtient ainsi un taux de désalumination global inférieur à 5% atomique pour la zéolithe MFI ainsi traitée c'est à dire que le traitement de désalumination retire, au plus, 5 % des atomes d'aluminium présents dans la charpente de la zéolithe. Ledit traitement confère à la MFI ainsi traitée des propriétés de sélectivité très améliorées. Cela se traduit par une inhibition surprenante des réactions secondaires indésirables telles que la réaction de dismutation et par une augmentation de la sélectivité en para-xylène. Cette nouvelle MFI modifiée conduit par ailleurs à des sélectivités vis-à-vis des réactions de désalkylation équivalentes à celles des catalyseurs basés sur la MFI non traitée. Les solides ainsi obtenus présentent des performances en isomérisation des C$_8$ aromatiques meilleures que celles des MFI de l'art antérieur.

La MFI utilisée dans le catalyseur de la présente invention est préparée à partir d'une MFI aussi bien synthétisée en milieu hydroxyde (brevet USP-3702886) que fluorure, en présence de structurant organique, (par exemple brevet US-A-5010048 et demandes de brevet EP-A-342075, EP-A-469151 et EP-A-472462) ou en absence de structurant organique (par exemple demande de brevet EP-A-500413).

La MFI sur laquelle est appliquée au moins un traitement par au moins une solution de sels d'acide fluorosilicique est obtenue de préférence directement à la synthèse avec le rapport Si/Al atomique désiré ou bien est obtenue par désalumination d'une MFI possédant un rapport Si/Al plus bas.

La modification de la sélectivité des MFI par au moins un traitement par au moins une solution de fluorosilicate se fait sur des MFI dont le rapport Si/Al atomique est compris entre 5 et 1000 et de préférence entre 5 et 500 et de manière encore plus préférée entre 5 et 250. Lesdites MFI sont sous forme Na$^+$, H$^+$, NH$_4^+$ ou bien contiennent encore le structurant organique qui a servi à leur synthèse ou sont sous toute forme mixte combinaisons des quatre formes précédemment citées, et de préférence sous la forme NH$_4^+$ et/ou la forme contenant le structurant organique.

Lorsque le traitement par au moins une solution de fluorosilicate se fait sur les formes NH4$^+$ des MFI, la mise sous forme NH$_4^+$ s'effectue selon les cas par les méthodes décrites ci-après :

- a) La MFI a été synthétisée en absence de structurant organique. Dans ce cas la MFI brute de synthèse contient dans sa microporosité du sodium qui est éliminé par plusieurs échanges ioniques par des solutions concentrées de nitrate d'ammonium (10N) qui permettent d'obtenir une teneur pondérale en sodium par rapport au poids de MFI sèche généralement inférieure à 2000 ppm, de préférence à 1000 ppm et, de manière encore plus préférée, à 500 ppm.
- b) La MFI a été synthétisée en présence d'un structurant organique. Dans ce cas la MFI brute de synthèse contient dans sa microporosité le structurant organique et du sodium. La MFI brute de synthèse subit dans un premier temps une calcination sous air de manière à éliminer de sa structure pratiquement tout le structurant organique qui

2

s'y trouve. Puis, elle est soumise à plusieurs échanges ioniques par des solutions concentrées de nitrate d'ammonium (10N) qui permettent d'obtenir une teneur pondérale en sodium par rapport au poids de MFI sèche généralement inférieure à 2000 ppm, de préférence à 1000 ppm et, de manière encore plus préférée, à 500 ppm.

Lorsque le traitement par au moins une solution de fluorosilicate se fait directement sur les formes MFI contenant le structurant organique aucun traitement préalable n'est réalisé sur la MFI. Le sodium résiduel contenu dans la microporosité de la zéolithe est alors pratiquement éliminé, après le dit traitement, en réalisant trois échanges ioniques par des solutions de nitrate d'ammonium.

Le fluorosilicate utilisé en tant qu'agent de désalumination et de source de silicium, permettant ainsi la réinsertion d'atomes de silicium au sein du réseau cristallin de la mordénite à la place des atomes d'aluminium extraits, peut être l'un des sels possédant la formule suivante: $M_{2/x}SiF_6$ où M est un cation métallique ou non métallique possédant la valence x. Les cations M peuvent donc être $NH_4^+$, des alkyls ammonium, $K^+$, $Na^+$, $Li^+$, $Ba^{2+}$, $Mg^{2+}$, $Cd^{2+}$, $Cu^+$, $Cu^{2+}$ $Ca^{2+}$, $Cs^+$, $Fe^{2+}$, $Co^{2+}$, $Pb^{2+}$, $Mn^{2+}$, $Rb^+$, $Ag^+$, $Sr^{2+}$, $Zn^{2+}$, $Tl^+$ et $H^+$. De préférence, l'hexafluorosilicate d'ammonium est utilisé, car il conduit à la formation de sel d'aluminium $(NH_4)_3AlF_6$ soluble dans l'eau qui peut être aisément éliminé. En général, la température de traitement est comprise entre 20 et 100°C, et de préférence entre 50 et 100 °C. Le traitement de la MFI s'effectue en présence d'acétate d'ammonium qui permet de tamponner le pH du milieu réactionnel à des valeurs, comprises entre 4 et 8, et de préférence entre 5,5 et 7, valeurs de pH pour lesquelles la zéolithe ne subit pas de destruction de la charpente par attaque acide directe.

Après l'étape d'addition de la solution de fluorosilicate à la suspension de MFI dans une solution d'acétate d'ammonium, le mélange réactionnel est laissé, sous agitation vigoureuse, à la température désirée pendant une période comprise entre 30 minutes et 48 heures, mais de préférence comprise entre 1 et 5 heures.

La MFI est ensuite filtrée à la température de la réaction et abondamment lavée à l'eau bouillante. Le volume d'eau bouillante utilisée pour effectuer ces lavages correspond à un v/p = 150 ml/g, (le rapport v/p est le rapport volume d'eau bouillante sur quantité de zéolithe sèche traitée. La zéolithe de type MFI est séchée sous flux d'air à 450°C durant 4 heures).

Après ce ou ces traitements, la MFI modifiée subit un traitement thermique destiné soit à décomposer les cations ammonium présents au sein du réseau et à obtenir ainsi la forme acide (H-M) de la MFI si le traitement a été réalisé sur la forme $NH_4^+$ de la MFI, soit est soumise à une calcination sous air pour éliminer le structurant organique suivie de plusieurs échanges ioniques par des solutions concentrées de nitrate d'ammonium (10N) qui permettent d'obtenir une teneur en pondérale en sodium par rapport au poids de MFI sèche généralement inférieure à 2000 ppm, de préférence à 1000 ppm et, de manière encore plus préférée, à 500 ppm dans le cas où le traitement est réalisé sur la MFI contenant le structurant organique ayant servi à sa synthèse.

La zéolithe peut ensuite être soumise au dépôt d'au moins un métal du groupe VIII de préférence choisi dans le groupe formé par le platine et le palladium, et mise en forme par toute technique connue de l'homme du métier. Elle peut en particulier être mélangée à une matrice, généralement amorphe, par exemple à une poudre humide de gel d'alumine. Le mélange est ensuite mis en forme, par exemple par extrusion au travers d'une filière. La teneur en MFI du mélange ainsi obtenu est généralement comprise entre 0,5 et 99,99% et avantageusement comprise entre 40 et 90% en poids par rapport au mélange (MFI + matrice). Elle est plus particulièrement comprise entre 10 et 60% et , de préférence, entre 15 et 40% en poids par rapport au mélange (MFI + matrice).

Dans la suite du texte on désignera par le terme support le mélange MFI + matrice.

La mise en forme peut être réalisée avec d'autres matrices que l'alumine, telles que par exemple la magnésie, la silice alumine, les argiles naturelles (kaolin, bentonite) et par d'autres techniques que l'extrusion, telles que le pastillage ou la dragéification.

Le métal hydrogénant du groupe VIII, de préférence Pt et /ou Pd, peut également être déposé sur le support par tout procédé connu de l'homme de l'art et permettant le dépôt du métal sur la MFI. On peut utiliser la technique d'échange cationique avec compétition où le compétiteur est de préférence le nitrate d'ammonium, le rapport de compétition étant au moins égal à environ 50 et avantageusement de 50 à 200. Dans le cas du platine ou du palladium, on utilise habituellement un complexe tétramine du platine ou un complexe tétramine du palladium : ces derniers se déposeront alors pratiquement en totalité sur la MFI. Cette technique d'échange cationique peut également être utilisée pour déposer directement le métal sur la poudre de MFI, avant son mélange éventuel avec une matrice.

Le dépôt du métal (ou des métaux) du groupe VIII est suivi en général d'une calcination sous air ou oxygène, usuellement entre 300 et 600°C durant 0,5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures ; de préférence on opère entre 350° et 550°C pendant 2 à 5 heures. La teneur en métal du groupe VIII (de préférence Pt et/ou Pd) déposé sur le catalyseur est obtenue à l'issue de l'échange est comprise habituellement entre 0,05 et 1,5%, de préférence entre 0,1 et 1%, en poids par rapport à l'ensemble du catalyseur.

On peut également déposer le platine et/ou le palladium non plus directement sur la MFI, mais sur le liant aluminique, avant ou après l'étape de mise en forme, en mettant en oeuvre un échange anionique avec de l'acide hexachloroplatinique, de l'acide hexachloropalladique et/ou du chlorure de palladium en présence d'un agent compétiteur, par exemple l'acide chlorhydrique. En général après le dépôt de platine et/ou de palladium, le catalyseur est comme précédemment soumis à une calcination puis réduit sous hydrogène comme indiqué ci-dessus.

Le catalyseur bifonctionnel obtenu par les procédures précédentes peut être mise en oeuvre notamment dans les réactions d'isomérisation d'une coupe C8 aromatique, comprenant par exemple soit uniquement un mélange de xylènes, soit un mélange de xylène(s) et d'éthylbenzène. L'isomérisation des alkyl-aromatiques, et en particulier des xylènes, revêt une importance commerciale considérable. C'est en général surtout le para-xylène qui est le produit le plus recherché, car il est notamment utilisé comme intermédiaire dans la fabrication des fibres polyesters. On préfère fabriquer le para-xylène en isomérisant le méta-xylène, qui lui peut être obtenu par isomérisation de l'ortho-xylène L'éthylbenzène qui est difficilement séparable par distillation du mélange des xylènes (les points d'ébullition des différents composés sont très proches), se trouve très souvent dans la charge d'isomérisation des hydrocarbures aromatiques en $C_8$.

Les conditions opératoires du procédé d'isomérisation d'une coupe $C_8$ aromatique effectuée en présence d'au moins un catalyseur selon l'invention sont les suivantes :

- température comprise entre 240 et 600°C, de préférence entre 350 et 510° C,
- pression comprise entre 0,05 et 10 MPa, de préférence entre 0,2 et 3 MPa,
- vitesse spatiale (pph, en masse de charge par unité de charge de catalyseur et par heure), comprise entre 0,5 et 200 $h^{-1}$, de préférence entre 2 et 100 $h^{-1}$,
- rapport molaire hydrogène sur hydrocarbure de la charge (H2/HC) compris entre 0.5 et 12, de préférence entre 2 et 6.

Les exemples qui suivent illustrent l'invention sans toutefois en limiter la portée ; ils sont donnés soit pour une charge formée de 80% d'ortho-xylène et de 20% d'éthylbenzène (% en poids), exemples 2,3,5 et 6, soit pour une charge formée de 80% de méta-xylène et de 20% d'éthylbenzène (% en poids), exemples 1 et 4.

### Exemple 1: Catalyseur C1 conforme à l'invention

La matière première utilisée est une MFI synthétisée en milieu fluorure qui possède un rapport Si/Al atomique global de 10,5 et une teneur pondérale en sodium par rapport au poids de MFI sèche d'environ 7 %.

La MFI est tout d'abord soumise à 3 échanges ioniques dans une solution de $NH_4NO_3$ 10N à environ 100°C pendant 4 heures, pour chaque échange. La teneur pondérale en sodium est ainsi inférieure à 50 ppm. La MFI forme $NH_4^+$ possède alors, un rapport Si/Al global de 10,5, un rapport Si/Al mesuré par XPS (ESCA), caractéristique de la surface externe des cristallites de la MFI de 10, un volume de maille élémentaire de 5,393 $nm^3$, un volume poreux à l'azote, mesuré à -196°C et à P/Po=0,19 de 0,22 $cm^3$ de liquide par gramme de MFI et une surface spécifique, mesurée par la méthode B.E.T de 410 $m^2/g$.

La MFI forme $NH_4$ obtenue précédemment est ensuite soumise au traitement par une solution d'hexafluorosilicate d'ammonium. Pour cela, 20 grammes de zéolithe sèche sont mis en suspension dans 400 ml d'une solution d'acétate d'ammonium (30 grammes d'acétate d'ammonium pour 200 ml d'eau distillée). Cette suspension est ensuite placée dans un tricol de 500 ml équipé d'un reflux, et d'une agitation mécanique. Le pH initial du milieu est de 7,05. La température est portée à 80°C. Puis à l'aide d'une pompe, 116 ml d'une solution d'hexafluorosilicate d'ammonium 0,5 M sont introduits. En fin d'addition, la quantité d'hexafluorosilicate d'ammonium injectée représente 0,29 mole pour 100 grammes de zéolithe sèche. Le système est maintenu à la température de la réaction durant encore 2 heures. Puis, la solution est refroidie à l'ambiante, la valeur mesurée du pH en fin de réaction est de 6,1. Le solide est alors filtré et lavé avec un volume d'eau distillée bouillante au moins égal à 6 litres, c'est à dire au moins 300 ml d'eau distillée par gramme de zéolithe sèche (V/P=300 ml/g). La zéolithe ainsi traitée est séchée à l'étuve à 105°C durant une nuit, puis calcinée sous air sec de manière à déammoniaquer la MFI et à obtenir la forme $H^+$. Le solide obtenu à l'issue des ces traitements est référencé HMFI-1.

Ce dernier est ensuite intimement mélangé avec de l'alumine sur laquelle 0,3% poids de platine sont dispersés. Le catalyseur constitué par le mélange HMFI-1 plus alumine contient 40% en poids d'alumine. La teneur pondérale en platine du catalyseur final (contenant HMFI-1) est donc d'environ 0,12% poids. Le catalyseur C1 ainsi fabriqué est ensuite mis en forme par pastillage, calciné sous air à 550°C durant 2 heures et réduit sous hydrogène à 500°C pendant 3 heures.

Les caractéristiques du solide obtenu (catalyseur C1) sont un rapport Si/Al atomique global égal à 10,5 et un rapport Si/Al de surface XPS égal à 29. Ce rapport de surface est mesuré par XPS (ESCA) et est caractéristique-de la surface externe des cristaux de la MFI traitée.

Le catalyseur C1 est alors testé en isomérisation du mélange méta-xylène (80% poids) et éthylbenzène (20% poids), à une température de 410°C, sous une pression de 1,2 MPa et un rapport molaire hydrogène sur hydrocarbures (H2/HC) de 4 environ.

Les performances du catalyseur C1 et des catalyseurs préparés dans les exemples suivants, reportées dans le tableau 1, sont définies par :

$$\text{Conversion de l'o-xylène (\%)} = \frac{\text{masse d'o-xylène dans la charge - masse d'o-xylène dans la recette}}{\text{masse d'o-xylène dans la charge}} \times 100$$

$$\text{Conversion du méta-xylène (\%)} =$$
$$\frac{\text{masse du méta-xylène dans la charge - masse du méta-xylène dans la recette}}{\text{masse du méta-xylène dans la charge}} \times 100$$

$$\text{Sélectivité en isomérisation (\%)} = \frac{\text{masse de m-xylène + masse de p-xylène}}{\text{masse de produits}} \times 100$$

$$\text{Approche à l'équilibre de l'o-xylène (AEQ-ox) (\%)} =$$
$$\frac{\text{nombre de moles d'o-xylène dans la recette}}{\text{nombre de moles d'o-xylène à l'équilibre thermodyynamique}} \times 100$$

$$\text{Approche à l'équilibre du méta-xylène (AEQ-mx) (\%)} =$$
$$\frac{\text{nombre de moles de méta-xylène dans la recette}}{\text{nombre de moles de méta-xylène à l'équilibre thermodynamique}} \times 100$$

$$\text{Rendement en C8 aromatique (\%)} = \frac{\text{masse de C8 aromatiques et de naphtènes dans la recette}}{\text{masse totale de C8 aromatiques dans la charge}} \times 100$$

$$\text{Sélectivité en dismutation (\%)} = \frac{\text{masse de triméthylbenzènes + masse de toulène + masse de benzène}}{\text{masse des produits}} \times 100$$

$$\text{Sélectivité en craquage (\%)} = \frac{\text{masse des gaz de C1 à C4}}{\text{masse des produits}} \times 100$$

### Exemple 2 : Catalyseur C2 conforme à l'invention

La matière première utilisée est une MFI sous forme $H^+$ qui possède, un rapport Si/Al atomique de 27, un volume de malle élémentaire de 5,341 $nm^3$, une teneur en sodium inférieure à 50 ppm poids, un volume poreux à l'azote, mesuré à - 196°C et à P/Po=0,19 de 0,19 $cm^3$ de liquide par gramme de MFI et une surface spécifique, mesurée par la méthode B.E.T de 439 $m^2$/g.

La MFI est tout d'abord soumise à 3 échanges ioniques dans une solution de $NH_4NO_3$ 10N à environ 100°C pendant 4 heures, pour chaque échange, de manière à l'obtenir sous forme NH4$^+$. Elle possède alors un rapport Si/Al mesuré par XPS (ESCA), caractéristique de la surface externe des cristallites de MFI égal à 26 et quasiment identique au rapport Si/Al global mesuré par fluorescence X (FX).

La MFI forme $NH_4^+$ obtenue précédemment est ensuite soumise au traitement par l'hexafluorosilicate d'ammonium. Pour cela, 20 grammes de zéolithe sèche sont mis en suspension dans 200 ml d'une solution d'acétate d'ammonium (20 grammes d'acétate d'ammonium pour 200 ml d'eau distillée). Cette suspension est ensuite placée dans un tricol de 500 ml équipé d'un reflux, et d'une agitation mécanique. Le pH initial du milieu est de 7,0. La température est portée à 80°C. Puis à l'aide d'une pompe 80 ml d'une solution d'hexafluorosilicate d'ammonium 0,3 M sont introduits. En fin d'addition, la quantité d'hexafluorosilicate d'ammonium injectée représente 0,12 mole pour 100 grammes de zéolithe sèche. Le système est maintenu à la température de la réaction durant encore 2 heures. Puis, la solution est refroidie à l'ambiante, la valeur mesurée du pH en fin de réaction est de 5,5. Le solide est alors filtré et lavé avec un volume d'eau distillée bouillante au moins égal à 6 litres, c'est-à-dire au moins 300 ml d'eau distillée par gramme de zéolithe sèche (V/P=300 ml/g). La zéolithe ainsi traitée est séchée à l'étuve à 105°C durant une nuit, puis calcinée sous air sec de manière à déammoniaquer la MFI et à obtenir la forme $H^+$. Le solide obtenu à l'issue des ces traitements est référencé HMFI-2.

Les étapes de mélange de la MFI et de l'alumine, de dispersion du platine, de mise en forme, de réduction du catalyseur et les catalyseur et les conditions de test d'isomérisation sont identiques à celles décrites dans l'exemple 1. Le catalyseur C2 est alors testé en isomérisation du mélange ortho-xylène (80% poids) et éthylbenzène (20% poids), à

5

une température de 410°C, sous une pression de 1,2 MPa et un rapport molaire hydrogène sur hydrocarbures (H2/HC) de 4 environ.

Les performances du catalyseur C2 ainsi obtenu et conforme à l'invention (dont la teneur en platine est d'environ 0,12% poids) sont reportées dans le tableau 2.

### Exemple 3 : Catalyseur C3 conforme à l'invention

La matière première utilisée est une MFI contenant le structurant organique ayant servi à sa synthèse, qui possède un rapport Si/Al atomique de 45 (mesuré par fluorescence X) et un volume de maille élémentaire de 5,367 nm$^3$, une teneur en sodium de 0,7 % poids.

La MFI décrite précédemment est soumise au traitement par l'hexafluorosilicate d'ammonium. Pour cela, 20 grammes de zéolithe sèche sont mis en suspension dans 200 ml d'une solution d'acétate d'ammonium (20 grammes d'acétate d'ammonium pour 200 ml d'eau distillée). Cette suspension est ensuite placée dans un tricol de 500 ml équipé d'un reflux, et d'une agitation mécanique. Le pH initial du milieu est de 6,8. La température est portée à 80°C. Puis à l'aide d'une pompe 55 ml d'une solution d'hexafluorosilicate d'ammonium 0,4 M sont introduits. En fin d'addition, la quantité d'hexafluorosilicate d'ammonium injectée représente 0,11 mole pour 100 grammes de zéolithe sèche. Le système est maintenu à la température de la réaction durant encore 2 heures. Puis, la solution est refroidie à l'ambiante, la valeur mesurée du pH en fin de réaction est de 5,7. Le solide est alors filtré et lavé avec un volume d'eau distillée bouillante au moins égal à 6 litres, c'est-à-dire au moins 300 ml d'eau distillée par gramme de zéolithe sèche (V/P=300 ml/g). La zéolithe ainsi traitée est séchée à l'étuve à 105°C durant une nuit, puis calcinée sous air sec de manière à éliminer totalement le structurant organique contenu dans sa porosité. Puis, la MFI est soumise à 3 échanges ioniques dans une solution de $NH_4NO_3$ 10N à environ 100°C pendant 4 heures, pour chaque échange, de manière à l'obtenir sous forme $NH_4^+$. Enfin, la MFI traitée et sous forme $NH_4^+$ est calcinée sous air sec de manière à la déammoniaquer et à obtenir la forme $H^+$. Le solide obtenu à l'issue des ces traitements est référencé HMFI-3.

Les étapes de mélange de la MFI et de l'alumine, de dispersion du platine, de mise en forme, de réduction du catalyseur sont identiques à celles décrites dans l'exemple 1.

Les caractéristiques du solide obtenu (catalyseur C3) sont un rapport Si/Al atomique global égal à 45 et un rapport Si/Al de surface XPS supérieur à 95. Ce rapport de surface est mesuré par XPS (ESCA) et est caractéristique de la surface externe des cristaux de la MFI traitée.

Le catalyseur C3 est alors testé en isomérisation du mélange ortho-xylène (80% poids) et éthylbenzène (20% poids), à une température de 410°C, sous une pression de 1,2 MPa et un rapport molaire hydrogène sur hydrocarbures (H2/HC) de 4 environ.

Les performances du catalyseur C3 ainsi obtenu et conforme à l'invention (dont la teneur en platine est d'environ 0,12% poids) sont reportées dans le tableau 2.

### Exemple 4 : Catalyseur NC1 non conforme à l'invention

La matière première utilisée dans cette exemple est la MFI sous forme $NH_4^+$ préparée dans l'exemple 1, mais non traitée selon l'invention.

Cette dernière est ensuite intimement mélangé avec de l'alumine sur laquelle 0,3% poids de platine sont dispersés. Le catalyseur constitué par le mélange MFI + alumine contient 40% en poids d'alumine. La teneur pondérale en platine du catalyseur final (contenant la MFI) est donc d'environ 0,12% poids.

Le catalyseur NC1 ainsi fabriqué est ensuite mis en forme par pastillage, calciné sous air à 550°C durant 2 heures et réduit sous hydrogène à 500°C pendant 3 heures.

Le catalyseur NC1 est alors testé en isomérisation du mélange méta-xylène (80% poids) et éthylbenzène (20% poids), à une température de 410°C, sous une pression de 1,2 MPa et un rapport molaire hydrogène sur hydrocarbures (H2/HC) de 4 environ.

Les performances du catalyseur NC1 sont reportées dans le tableau 1.

### Exemple 5 : Catalyseur NC2 non conforme à l'invention

La matière première utilisée dans cette exemple est la MFI sous forme $H^+$ utilisée dans l'exemple 2 et non traitée selon l'invention.

Les étapes de mise sous forme $NH_4^+$ de la MFI, de mélange de la MFI et de l'alumine, de dispersion du platine, de mise en forme, de réduction du catalyseur sont identiques à celles décrites dans l'exemple 1.

Le catalyseur NC2 est alors testé en isomérisation du mélange ortho-xylène (80% poids) et éthylbenzène (20% poids), à une température de 410°C, sous une pression de 1,2 MPa et un rapport molaire hydrogène sur hydrocarbures (H2/HC) de 4 environ.

Les performances du catalyseur NC2 ainsi obtenu et non conforme à l'invention (dont la teneur en platine est d'environ 0,12%) sont reportées dans le tableau 2.

### Exemple 6 : Catalyseur NC3 non conforme à l'invention

La matière première utilisée dans cet exemple non conforme à l'invention est une MFI contenant le structurant organique ayant servi à sa synthèse, qui possède un rapport Si/Al atomique de 45 mais n'est pas traitée selon l'invention.

La zéolithe est séchée à l'étuve à 105°C durant une nuit, puis calcinée sous air sec de manière à éliminer totalement le structurant organique contenu dans sa porosité. Puis, la MFI est soumise à 3 échanges ioniques dans une solution de $NH_4NO_3$ 10N à environ 100°C pendant 4 heures, pour chaque échange de manière à l'obtenir sous forme $NH_4^+$. Enfin, la MFI traitée et sous forme $NH_4^+$ est calcinée sous air sec de manière à la déammoniaquer et à obtenir la forme $H^+$.

Les étapes de mélange de la MFI et de l'alumine, de dispersion du platine, de mise en forme, de réduction du catalyseur sont identiques à celles décrites dans l'exemple 1.

Le catalyseur NC3 est alors testé en isomérisation du mélange ortho-xylène (80% poids) et éthylbenzène (20% poids), à une température de 410°C, sous une pression de 1,2 MPa et un rapport molaire hydrogène sur hydrocarbures (H2/HC) de 4 environ.

Les performances du catalyseur NC3 ainsi obtenu et non conforme à l'invention (dont la teneur en platine est d'environ 0,12% poids) sont reportées dans le tableau 2.

### Effet des traitements des MFI par l'hexafluorosilicate d'ammonium, sur les sélectivités à Iso-approche à l'équilibre

Le tableau 1 décrit les performances des catalyseurs C1, et NC1 et le tableau 2 celles des catalyseurs C2, C3, NC2 et NC3, préparés selon les modes opératoires décrits précédemment. L'effet du traitement à l'hexafluorosilicate d'ammonium sur les sélectivités est particulièrement mis en évidence.

Tableau 1

| Charges | 80% (poids) méta-xylène + 20% (poids) éthylbenzène | |
|---|---|---|
| Catalyseurs | NC1 | C1 |
| Exemples | 4 | 1 |
| % AEQ méta-xylène | 95 | 95 |
| % Rdt $C_8$ (aromatiques + naphtènes) | 68 | 82 |
| % Dismutation | 17 | 8 |
| % Désalkylation | 16 | 14 |
| % Craquage | 7 | 3 |
| Rapport para-xylène/méta-xylène | 0,43 | 0,65 |

Tableau 2

| Charges | 80% (poids) ortho-xylène + 20% (poids) éthylbenzène | | | |
|---|---|---|---|---|
| Catalyseurs | NC2 | C2 | NC3 | C3 |
| Exemples | 5 | 2 | 6 | 3 |
| % AEQ o-xylène | 97 | 97 | 96,5 | 96,5 |

Tableau 2 (suite)

| Charges | 80% (poids) ortho-xylène + 20% (poids) éthylbenzène | | | |
|---|---|---|---|---|
| % Rdt C$_8$ (aromatiques + naphtènes) | 67 | 80 | 70 | 79 |
| % Dismutation | 14 | 6 | 16 | 9 |
| % Désalkylation | 13 | 12 | 12 | 11 |
| % Craquage | 4,5 | 1,5 | 2 | 1 |

Dans les tableaux 1 et 2 les valeurs des vitesses spatiales (pph) en (heure)-1 sont ajustées de manière à obtenir des % AEQ, en ortho-xylène et méta-xylène, comparables.

Le catalyseur C1 conforme à l'invention est plus performant que le catalyseur NC1 de l'art antérieur. En effet, à iso-approche à l'équilibre du méta-xylène, le taux des réactions secondaires de dismutation, conduisant à la formation, en outre, de triméthylbenzènes, est fortement réduit dans le cas du catalyseur conforme à l'invention C1. D'autre part, il apparaît que le catalyseur traité selon l'invention (C1) conduit à un taux de craquage plus faible que le catalyseur de l'art antérieur, NC1, mais surtout à une plus grande sélectivité en isomère para-xylène. Il s'ensuit que le rendement en C8 aromatiques + naphtènes obtenu sur ce catalyseur est supérieur à celui du catalyseur de l'art antérieur. Le catalyseur traité selon l'invention possède donc le double avantage de réduire le taux des réactions indésirables (réactions de dismutation et de craquage) tout en augmentant la sélectivité en para-xylène formé.

Les catalyseurs C2, et C3 conformes à l'invention sont plus performants que les catalyseurs NC2 et NC3 de l'art antérieur. En effet, à iso-approche à l'équilibre de l'o-xylène, le rendement en isomérisation des C8 aromatiques + naphtènes obtenus sur les catalyseurs C2 et C3 est supérieur à celui des catalyseurs NC2 et NC3. D'autre part, dans le cas des MFI traités selon l'invention (catalyseurs C2, C3) la réaction secondaire de dismutation conduisant à la formation de triméthylbenzènes est fortement inhibée par rapport à ce que l'on obtient en présence de MFI non sélectivée (catalyseurs C2 et C3). De même, le taux des réactions de craquage est fortement diminué sur les catalyseurs comportant les MFI ayant subi le traitement par au moins une solution aqueuse d'hexafluorosilicate d'ammonium selon l'invention. Par ailleurs, on peut observer que le taux des réactions de désalkylation n'est pas modifié par le traitement effectué selon l'invention.

L'analyse par fluorescence X, des MFI; HMFI-1, HMFI-2 et HMFI-3 traitées selon l'invention, montre que les rapports Si/Al globaux (atomiques) ainsi mesurés sont sensiblement identiques aux rapports Si/Al globaux (atomiques) des MFI de départ avant traitement. Par contre on note une augmentation des rapports Si/Al mesurés par XPS (ESCA) après traitement selon l'invention.

## Revendications

1. Catalyseur comprenant une matrice, une zéolithe de type MFI et au moins un élément du groupe VIII, caractérisé en ce que les cristaux de la zéolithe de type MFI sont désaluminés sur leur surface externe par au moins un traitement avec au moins une solution d'un fluorosilicate d'un cation choisi dans le groupe formé par NH$_4^+$, les alkyls ammonium, K$^+$, Na$^+$, Li$^+$, Ba$^{2+}$, Mg$^{2+}$, Cd$^{2+}$, Cu$^+$, Cu$^{2+}$ Ca$^{2+}$, Cs$^+$, Fe$^{2+}$, Co$^{2+}$, Pb$^{2+}$, Mn$^{2+}$, Rb$^+$, Ag$^+$, Sr$^{2+}$, Zn$^{2+}$, Tl$^+$ et H$^+$, le taux de désalumination global de ladite zéolithe ainsi traitée est inférieur à 5% atomique.

2. Catalyseur selon la revendication 1 dans lequel la zéolithe MFI est traitée avec une solution d'hexafluorosilicate d'ammonium.

3. Catalyseur selon l'une des revendications précédentes,dans lequel la zéolithe MFI à traiter présente un rapport Si/Al atomique compris entre 5 et 1000.

4. Catalyseur selon l'une des revendications précédentes, dans lequel le traitement est effectué entre 20 et 100°C et à un pH compris entre 4 et 8.

5. Catalyseur selon l'une des revendications précédentes, dans lequel l'élément du groupe VIII est choisi dans le groupe formé par le platine et le palladium.

6. Catalyseur selon l'une des revendications précédentes, dans lequel l'élément du groupe VIII est déposé sur la zéolithe.

**7.** Catalyseur selon l'une des revendications précédentes, dans lequel l'élément du groupe VIII est déposé sur la matrice.

**8.** Catalyseur selon l'une des revendications précédentes, dans lequel la matrice est choisie dans le groupe formé par l'alumine, la magnésie, la silice alumine, les argiles naturelles, leurs mélanges.

**9.** Catalyseur selon l'une des revendications précédentes, dans lequel les cristaux de la zéolithe de type MFI sont désaluminés sur leur surface externe par au moins un traitement avec au moins une solution d'un fluorosilicate, le temps de contact entre la zéolithe et la solution de fluorosilicate étant de 30 mn à 5 h.

**10.** Procédé d'isomérisation de coupes $C_8$ aromatique avec un catalyseur selon l'une des revendications 1 à 9, dans lequel la température est comprise entre 240 et 600 °C, la pression entre 0,05 et 10 MPa, la vitesse spatiale entre 0,5 et $200h^{-1}$, la rapport molaire $H_2/HC$ entre 0,5 et 12.

**11.** Procédé selon la revendication 10, dans lequel la coupe $C_8$ traitée est un mélange de xylènes.

**12.** Procédé selon la revendication 11, dans lequel la coupe traitée contient également de l'éthylbenzène.

**Claims**

**1.** A catalyst comprising a matrix, a MFI type zeolite and at least one group VIII element, characterised in that the crystals of the MFI type zeolite are desaluminated on their outer surface by at least one treatment with at least one solution of a fluorosilicate of a cation selected from the group formed by $NH_4^+$, the alkylammoniums, $K^+$, $Na^+$, $Li^+$, $Ba^{2+}$, $Mg^{2+}$, $Cd^{2+}$, $Cu^+$, $Cu^{2+}$, $Ca^{2+}$, $Cs^+$, $Fe^{2+}$, $Co^{2+}$, $Pb^{2+}$, $Mn^{2+}$, $Rb^+$, $Ag^+$, $Sr^{2+}$, $Zn^{2+}$, $Tl^+$ and $H^+$, the global rate of desalumination of said zeolite thus treated is less than 5 atomic per cent.

**2.** A catalyst according to Claim 1, in which the MFI zeolite is treated with a solution of ammonium hexafluorosilicate solution.

**3.** A catalyst according to one of the preceding claims, in which the MFI zeolite to be treated has an atomic Si/Al ratio of between 5 and 1000.

**4.** A catalyst according to one of the preceding claims, in which the treatment is carried out between 20 and 100°C and at a pH of between 4 and 8.

**5.** A catalyst according to one of the preceding claims, in which the group VIII element is selected from the group formed by platinum and palladium.

**6.** A catalyst according to one of the preceding claims, in which the group VIII element is deposited on the zeolite.

**7.** A catalyst according to one of the preceding claims, in which the group VIII element is deposited on the matrix.

**8.** A catalyst according to one of the preceding claims, in which the matrix is selected from the group formed by alumina, magnesium, silica alumina, natural clays, their mixtures.

**9.** A catalyst according to one of the preceding claims, in which the crystals of MFI type zeolite are desaluminated on their outer surface by at least one treatment with at least one solution of a fluorosilicate, the zeolite and the solution of a fluorosilicate being in contact from 30 min to 5 hours.

**10.** A process for the isomerisation of $C_8$ aromatic cuts with a catalyst according to claim 1 to 9, in which the temperature is between 240 and 600°C, the pressure is between 0.05 and 10 MPa, the spatial speed is between 0.5 and $200^{\,h-1}$, and the $H_2/HC$ molar ratio is between 0.5 and 12.

**11.** A process according to claim 10, in which the cut treated is a mixture of xylene.

**12.** A process according to claim 11, in which the cut treated also contains ethyl benzene.

**Patentansprüche**

1. Katalysator umfassend eine Matrix, einen Zeolith vom MFI-Typ und wenigstens ein Element der Gruppe VIII, dadurch gekennzeichnet, daß die Kristalle des Zeoliths vom MFI-Typ auf ihrer Außenfläche durch wenigstens eine Behandlung mit wenigstens einer Lösung eines Fluorsilikats eines Cations entaluminisiert werden, das gewählt ist aus der Gruppe, die durch $NH_4+$, die Ammoniumalkyle, $K^+$, $Na^+$, $Li^+$, $Ba^{2+}$, $Mg^{2+}$, $Cd^{2+}$, $Cu^+$, $Cu^{2+}$ $Ca^{2+}$, $Cs^+$, $Fe^{2+}$, $Co^{2+}$, $Pb^{2+}$, $Mn^{2+}$, $Rb^+$, $Ag^+$, $Sr^{2+}$, $Zn^{2+}$, $Tl^+$ und $H^+$ gebildet ist, wobei das globale Entaluminisierungsverhältnis dieses so behandelten Zeoliths weniger als 5 Atom-% beträgt.

2. Katalysator nach Anspruch 1, bei dem der MFI-Zeolith mit einer Ammonium-Hexafluorsilikatlösung behandelt wird.

3. Katalysator nach einem der vorhergehenden Ansprüche, bei dem der zu behandelnde MFI-Zeolith ein Si/Al Atomverhältnis zwischen 5 und 1000 aufweist.

4. Katalysator nach einem der vorhergehenden Ansprüche, bei dem die Behandlung zwischen 20 und 100°C und bei einem pH-Wert zwischen 4 und 8 durchgeführt wird.

5. Katalysator nach einem der vorhergehenden Ansprüche, bei dem das Element der Gruppe VIII gewählt ist aus der durch Platin und Palladium gebildeten Gruppe.

6. Katalysator nach einem der vorhergehenden Ansprüche, bei dem das Element der Gruppe VIII auf den Zeolith abgeschieden ist.

7. Katalysator nach einem der vorhergehenden Ansprüche, bei dem das Element der Gruppe VIII auf die Matrix abgeschieden ist.

8. Katalysator nach einem der vorhergehenden Ansprüche, bei dem die Matrix gewählt ist aus der durch Aluminiumoxid, Magnesiumoxid, Siliciumoxid-Aluminiumoxid, die natürlichen Tone und deren Gemische gebildeten Gruppe.

9. Katalysator nach einem der vorhergehenden Ansprüche, bei dem die Kristalle des Zeoliths vom MFI-Typ auf ihrer Außenfläche durch wenigstens eine Behandlung mit wenigstens einer Fluorsilikatlösung entaluminisiert sind, wobei die Kontaktzeit zwischen dem Zeolith und der Fluorsilikatlösung 30 Minuten bis 5 Stunden beträgt.

10. Verfahren zur Isomerisierung von aromatischen $C_8$-Fraktionen mit einem Katalysator nach einem der Ansprüche 1 bis 9, bei dem die Temperatur zwischen 240 und 600°C, der Druck zwischen 0,05 und 10 MPa, die Raumgeschwindigkeit zwischen 0,5 und 200$h^{-1}$ und das Molverhältnis $H_2$/HC zwischen 0,5 und 12 liegt.

11. Verfahren nach Anspruch 10, bei dem die behandelte $C_8$-Fraktion ein Gemisch aus Xylenen ist.

12. Verfahren nach Anspruch 11, bei dem die behandelte Fraktion auch Ethylbenzen enthält.